Europäisches Patentamt

European Patent Office

Office européen des brevets

(11) Numéro de publication: **0 162 790**
**B2**

(12) **NOUVEAU FASCICULE DE BREVET EUROPEEN**

(45) Date de publication du nouveau fascicule du brevet: **09.01.91**

(21) Numéro de dépôt: **85420072.2**

(22) Date de dépôt: **24.04.85**

(51) Int. Cl.⁵: **C 07 C 29/10**, C 07 C 31/20, C 07 D 301/32

(54) **Procédé de séparation de l'éthylèneglycol sous forme concentrée dans un procédé de fabrication d'oxyde d'éthylène.**

(30) Priorité: **15.05.84 FR 8407695**

(43) Date de publication de la demande:
**27.11.85 Bulletin 85/48**

(45) Mention de la délivrance du brevet:
**12.10.88 Bulletin 88/41**

(45) Mention de la decision concernant l'opposition:
**09.01.91 Bulletin 91/02**

(84) Etats contractants désignés:
**BE DE FR GB IT NL SE**

(56) Documents cités:
**US-A-3 904 656**

(73) Titulaire: **ATOCHEM**
**4 & 8, Cours Michelet La Défense 10**
**F-92800 Puteaux (FR)**

(72) Inventeur: **Neel, Henry**
**7 Boulevard François Ier**
**F-76600 Le Havre (FR)**
Inventeur: **Delannoy, Francis**
**61 rue Ampère**
**F-69310 Pierre-Bénite (FR)**

EP 0 162 790 B2

**Description**

La présente invention concerne un procédé pour séparer l'éthylèneglycol dans un procédé de fabrication d'oxyde d'éthylène par oxydation catalytique en phase gazeuse de l'éthylène par l'oxygène.

Les deux premières étapes habituellement adoptées pour isoler l'oxyde d'éthylène à partir du mélange gazeux résultant d'une telle réaction sont:

— une absorption consistant à mettre en contact le mélange gazeux avec de l'eau pour former une solution aqueuse diluée d'oxyde d'éthylène renfermant des gaz à l'état dissous et notamment du dioxyde de carbone.

— une désorption consistant à soumettre la solution aqueuse d'oxyde d'éthylène résultant de l'étape d'absorption à l'action de la vapeur d'eau dans une colonne délivrant en pied un courant aqueux pratiquement débarrassé d'oxyde d'éthylène servant, après refroidissement de fluide d'absorption dans la première des deux étapes décrites.

Ce courant aqueux, désigné dans ce qui suit par l'expression eau glycolée, s'enrichirait progressivement et continuellement en éthylèneglycol formé par hydratation de l'oxyde d'éthylène dans les conditions auxquelles ce composé est soumis, particulièrement lors de l'étape de desorption, s'il n'en était évacué une partie pour limiter la concentration d'éthylèneglycol à une valeur n'excédant généralement pas 10% en poids environ.

L'économie du procédé de fabrication de l'oxyde d'éthylène, y compris les problèmes de pollution, réclame que soit récupéré l'éthylèneglycol contenu dans la partie d'eau glycolée évacuée.

En pratique, l'eau glycolée renferme entre autres, à coté de l'éthylèneglycol, d'autres glycols comme le diéthylèneglycol ou le triéthylèneglycol, des impuretés génanntes telles que celles signalées dans le brevet des Etats-Unis d'Amérique n° 3 904 656 et des sels, principalement des carbonates de sodium ou de potassium et des sels de sodium ou de potassium d'acides carboxyliques. La présence de ces sels est due à l'addition d'hydroxyde de sodium ou de potassium à l'eau glycolée qui engendrerait sinon des phénomènes de corrosion, son pH étant rendu normalement acide par le dioxyde de carbone et les acides carboxyliques qu'elle renferme.

Les procédés connus préconisent, pour séparer l'éthylèneglycol du courant d'eau glycolée évacuée du cycle, des traitements complexes et coûteux.

C'est ainsi que le brevet des Etats-Unis d'Amérique n° 3 904 656 préconise le passage de la solution glycolée évacuée sur des résines échangeuses d'ions et sur du charbon actif avant de diriger le courant aqueux résultant de ce traitement et contenant l'éthylèneglycol vers une étape d'un procédé de fabrication d'éthylèneglycol à partir d'oxyde d'éthylene.

Le brevet français n° 2 246 527 décrit pour sa part un traitement de l'eau glycolée évacuée par osmose inverse délivrant d'une part un courant aqueux renfermant environ 60% de l'éthylèneglycol initialement contenu en elle et pratiquement plus de sels, d'autre part un courant aqueux renfermant pratiquement la totalité des sels et le complément de l'éthylèneglycol. Le premier des deux courants aqueux est introduit dans une installation de production de glycol. L'éthylèneglycol contenu dans le second courant aqueux n'est pas récupéré.

Selon le brevet français n° 2 295 938 l'éthylèneglycol est séparé à partir de la solution glycolée évacuée par mise en contact de celle-ci avec des échangeurs de cations et des échangeurs d'anions préalablement à la séparation des glycols par distillation.

Aucun des procédés connus ne réalise la séparation de l'éthylèneglycol sous forme concentrée, directement à partir de l'eau glycolée évacuée et sans séparation préalable des sels qu'elle contient.

C'est le problème que résout le procédé de l'invention.

Le procédé de l'invention consiste à séparer l'éthylèneglycol à partir d'eau glycolée issue au bas de la colonne de désorption de l'oxyde d'éthylène dans un procédé de fabrication d'oxyde d'éthylène par oxydation catalytique en phase vapeur de l'éthylène par l'oxygène, et contenant jusqu'à 10% en poids d'éthylèneglycol et des sels de sodium ou de potassium résultant de l'addition d'hydroxyde de sodium ou de potassium à l'eau glycolée pour prévenir la corrosion par l'acide carbonique ou les acides carboxyliques qu'elle peut contenir, caractérisé en ce que l'éthylèneglycol est séparé sous forme d'un mélange avec de l'eau renfermant 40% à 90% en poids d'éthylèneglycol et les sels obtenu au bas de la colonne directement alimentée à son niveau supérieur par l'eau glycolée qui comporte au-dessous de ce niveau jusqu'à 12 plateaux théoriques, qui fonctionne à une pression absolue moyenne au moins égale à celle de la colonne de désorption de l'oxyde d'éthylène et inférieure à 6 bars, qui est pourvue en calories par échange thermique indirect, qui délivre en tête un courant gazeux essentiellement constitué de vapeur d'eau qui est introduit dans la colonne de désorption de l'oxyde d'éthylène d'où est issue l'eau glycolée, pour servir de courant de désorption, la quantité d'énergie thermique à fournir n'étant pas supérieure à celle fournie lorsque le procédé de l'invention n'est pas appliqué et l'éthylèneglycol étant isolé de façon connue du mélange recueilli au bas de la colonne par élimination de l'eau et évaporation.

La fraction d'eau glycolée à traiter est introduite dans la colonne de séparation de l'éthylèneglycol dans les conditions de température et de pression qui sont celles ou qui sont voisines de celles dans lesquelles se trouve l'eau glycolée sortant de la colonne de désorption.

La présence des sels communément présents dans l'eau glycolée ne gêne en rien la réalisation du procédé selon l'invention.

Comme indiqué la pression moyenne absolue dans la colonne du procédé de l'invention est au moins égale à celle de la colonne de désorption de l'oxyde d'éthylène. Elle est choisie inférieure à 6 bars.

La température dans la colonne dépend de cette pression et de la concentration désirée dans le courant issu au bas de celle-ci et comprenant l'éthylèneglycol séparé de la fraction d'eau glycolée traitée.

Les calories sont fournies à la colonne à l'aide par exemple de vapeur d'eau ou d'un fluide caloporteur.

Le courant issu au bas de la colonne et qui contient l'éthylèneglycol peut être traité, ultérieurement à son obtention, de toute manière conventionnelle et connue pour isoler l'éthylèneglycol, par exemple par élimination de l'eau puis distillation de l'éthylèneglycol dans un évaporateur à film. Il peut, dans le même but, être réuni avant isolement à un courant renfermant de l'éthylèneglycol dans un procédé de fabrication de ce composé à partir d'oxyde d'éthylène.

Les exemples suivants, donnés à titre non limitatif, illustrent l'invention:

### Exemple 1

Dans un procédé de fabrication d'oxyde d'éthylène par oxydation catalytique en phase vapeur de l'éthylène par l'oxygène, qui comporte un système d'isolement de l'oxyde d'éthylène comportant une colonne d'absorption par l'eau de l'oxyde d'éthylène contenu dans le mélange gazeux issu de la zone de catalyse et une colonne de désorption par la vapeur d'eau de l'oxyde d'éthylène ainsi absorbé, 387,1 kg/h d'un courant aqueux contenant, en poids, 94,4% d'eau, 2,54% d'oxyde d'éthylène et 3% d'éthylèneglycol, sont introduits à une température de 108°C dans la colonne de désorption comportant 5 plateaux théoriques. En haut de cette colonne sortent 23,9 kg/h d'un mélange gazeux à une température de 97°C et à une pression absolue de 1,2 bars, qui renferment, en poids, 58% d'eau, 40,5% d'oxyde d'éthylène à côté principalement de gaz tels que le dioxyde de carbone initialement présents à l'état dissous dans la solution aqueuse diluée d'oxyde d'éthylène introduite dans la colonne de désorption.

Le courant d'eau glycolée issu à 109°C au bas de la colonne de désorption à raison de 376,2 kg/h renferme, en poids 3,1% d'éthylèneglycol et moins de 0,01% d'oxyde d'éthylène. Il est alors divisé en deux parties dont la première, soit 362,5 kg/h sert, après refroidissement, de courant d'absorption de l'oxyde d'éthylène dans la première des deux colonnes citées en début d'exemple.

La seconde partie, soit 13,74 kg/h est introduite à la température de 109°C, au niveau supérieur d'une colonne de traitement d'eau glycolée comportant 2 plateaux théoriques.

Le courant gazeux issu en haut de cette colonne, a une température de 109,5°C, à une pression absolue de 1,4 bars et au débit de 13,07 kg/h est pratiquement constitué de vapeur d'eau et est introduit dans la colonne d'où est issue l'eau glycolée pour y servir de fluide de désorption de l'oxyde d'éthylène.

Le courant aqueux, issu au bas de la colonne de traitement d'eau glycolée au débit de 0,67 kg/h et à une température de 120,5°C renferme 60% en poids d'éthylèneglycol.

Un tel résultat de concentration de l'éthylèneglycol et d'efficacité de récupération de l'oxyde d'éthylène demande que soient fournies 1,5 thermies/heure à la colonne de désorption de l'éthylèneglycol et 7 thermies/heure à la colonne de traitement de l'eau glycolée.

A efficacité de désorption de l'oxyde d'éthylène identique, une quantité équivalente d'énergie calorifique ne conduit qu'à l'obtention de l'éthylèneglycol sous forme d'une solution aqueuse diluée ne contenant que 3,1% d'éthylèneglycol en poids lorsque le procédé de l'invention n'est pas réalisé.

### Exemple 2

En opérant la désorption de l'oxyde d'éthylène comme dans l'exemple 1 mais en soutirant en tête, à une température de 137,5°C et à une pression absolue de 5 bars, 18,9 kg/h d'un courant gazeux renfermant, en poids, 51% d'oxyde d'éthylène et 47% d'eau, 383,9 kg/h d'eau glycolée renfermant 3,3% en poids d'éthylèneglycol et moins de 0,01% en poids d'oxyde d'éthylène sont soutirés au bas de la colonne à une température de 153°C. 16,41 kg/h d'eau glycolée sont introduits à cette température dans la même colonne que dans l'exemple 1 pour être soumis au traitement selon l'invention. 15,74 kg/h d'un courant gazeux essentiellement constitué de vapeur d'eau sont issus en tête de la colonne, à une température de 154°C et à une pression absolue de 5,2 bars, puis sont introduits dans la colonne de désorption de l'oxyde d'éthylène.

En bas de la colonne, l'éthylèneglycol est recueilli à 166°C sous forme de 0,67 kg/h d'un courant aqueux renfermant, en poids, 60% d'éthylèneglycol.

Ce résultat est atteint sans qu'il ait été nécessaire de fournir au système une quantité d'énergie calorifique supérieure à celle qu'aurait nécessité le système n'incluant pas le traitement selon l'invention et assurant la même efficacité de désorption de l'oxyde d'éthylène mais seulement l'obtention d'une solution aqueuse diluée d'éthylèneglycol ne renfermant que 3,3% en poids de ce produit.

### Exemple 3

Le courant aqueux de l'exemple 1 à partir duquel l'oxyde d'éthylène doit être désorbé est introduit au même débit, à la même température et dans la même colonne que dans l'exemple 1.

23,2 kg/h d'un courant gazeux renfermant, en poids, 42% d'oxyde d'éthylène et 56.5% d'eau sont issus de la tête de la colonne à une pression absolue de 3 bars et à une température de 124°C.

En pied de colonne sortent 395,4 kg/h d'eau glycolée renfermant, en poids, 3,2% d'éthylène-glycol, à une température de 135°C.

31,9 kg/h d'eau glycolée sont introduits à cette température dans la même colonne de traitement selon l'invention que dans l'exemple 1.

En tête de cette colonne sont issus 31,46 kg/h d'un courant gazeux essentiellement constitué de vapeur d'eau, à une température de 136°C qui sont introduits à cette température dans la colonne de désorption de l'oxyde d'éthylène.

L'éthylèneglycol est récupéré avec de l'eau, en pied de la colonne de traitement de l'eau glycolée à une température de 180°C, sous forme d'un courant renfermant 90% en poids d'éthylène-glycol, au débit de 0,445 kg/h.

La quantité d'énergie thermique fournie est équivalente à celle assurant la même efficacité de désorption de l'oxyde d'éthylène dans un système ne comportant pas le traitement de l'eau glycolée selon l'invention et délivrant l'éthylène-glycol sous forme de solution aqueuse ne renfermant que 3,2% en poids d'éthylèneglycol.

Exemple 4

En opérant comme dans l'exemple 1 mais en introduisant dans la colonne de désorption de l'oxyde d'éthylène, à une température de 108°C, 403,7 kg/h d'un courant aqueux renfermant, en poids, à côté de 90,5% d'eau, 2,44% d'oxyde d'éthylène et 7% d'éthylèneglycol, 17,9 kg/h d'un courant gazeux, à une température de 120°C et à une pression absolue de 3 bars sont issus en tête de la colonne. Ils contiennent, en poids, 54% d'oxyde d'éthylène et 44% d'eau.

En pied de la colonne sont soutirés 393,4 kg/h d'eau glycolée, à une température de 135,5°C, qui contiennent 7,3% en poids d'éthylèneglycol.

8,1 kg/h d'eau glycolée sont traités dans la colonne de traitement de l'eau glycolée de l'exemple 1. Sont soutirés de cette colonne, en tête 7,65 kg/h d'un courant gazeux à la température de 137°C et à la pression de 3,2 bars. Ce courant, pratiquement constitué de vapeur d'eau, est introduit dans la colonne de désorption de l'oxyde d'éthylène.

En pied de la colonne de traitement de l'eau glycolée est recueilli, à 180°C et à raison de 0,445 kg/h, un courant aqueux renfermant, en poids, 90% d'éthylèneglycol.

Pour qu'un tel résultat soit obtenu il est fourni une quantité d'énergie calorifique équivalente à celle qui permettrait d'obtenir la même efficacité de récupération de l'oxyde d'éthylène sans mise en oeuvre de l'invention, mais seulement une solution aqueuse diluée d'éthylèneglycol ne contenant que 7,3% d'éthylèneglycol en poids.

Revendications

1. Procédé de séparation de l'éthylèneglycol à partir d'eau glycolée issue au bas de la colonne de désorption de l'oxyde d'éthylène dans un procédé de fabrication d'oxyde d'éthylène par oxydation catalytique en phase vapeur de l'éthylène par l'oxygène, et contenant jusqu'à 10% en poids d'éthylèneglycol et des sels de sodium ou de potassium résultant de l'addition d'hydroxyde de sodium ou de potassium à l'eau glycolée pour prévenir la corrosion par l'acide carbonique ou les acides carboxyliques qu'elle peut contenir, caractérisé en ce que l'éthylèneglycol est séparé sous forme d'un mélange avec de l'eau renfermant 40% à 90% en poids d'éthylèneglycol et les sels obtenu au bas d'une colonne directement alimentée à son niveau supérieur par l'eau glycolée, qui comporte au-dessous de ce niveau jusqu'à 12 plateaux théoriques, qui fonctionne à une pression absolue moyenne au moins égale à celle de la colonne de désorption de l'oxyde d'éthylène et inférieure à 6 bars, qui est pourvue en calories par échange thermique indirect, qui délivre en tête un courant gazeux essentiellement constitué de vapeur d'eau qui est introduit dans la colonne de désorption de l'oxyde d'éthylène d'où est issue l'eau glycolée pour servir de courant de désorption, la quantité d'énergie thermique à fournir n'étant pas supérieure à celle fournie lorsque le procédé de l'invention n'est pas appliqué et l'éthylèneglycol étant isolé de façon connue du mélange recueilli au bas de la colonne par élimination de l'eau et évaporation.

2. Procédé selon la revendication 1, caractérisé en ce que l'eau glycolée, est introduite dans la colonne de séparation de l'éthylèneglycol à la température à laquelle elle est issue de la colonne de désorption de l'oxyde d'éthylène.

Patentansprüche

1. Verfahren zur Abtrennung von Ethylenglykol aus glykolhaltigem Wasser, das aus dem unteren Ende einer Ethylenoxid-Desorptionskolonne eines Verfahrens zur Herstellung von Ethylenoxid durch katalytische Oxidation von Ethylen durch Sauerstoff in der Dampfphase stammt und bis zu 10 Gew.% Ethylenglykol und Natrium- oder Kaliumsalze enthält, die von der Natrium- oder Kaliumhydroxidzugabe zum glykolhaltigen Wasser zum Schutz vor Korrosion durch Kohlensäure oder Carbonsäuren, die es enthalten kann, herrühren, dadurch gekennzeichnet, daß das Ethylenglykol abgetrennt wird in Form eines 40 bis 90 Gew.% Ethylenglykol und die Salze umfassenden, am unteren Ende einer Kolonne erhaltenen Gemischs mit Wasser, welcher direkt an ihrem oberen Ende glykolhaltiges Wasser zugeführt wird und die unter dieser Ebene bis zu 12 theoretische Böden enthält, bei einem mittleren absoluten Druck von wenigstens gleich dem der Ethylenoxid-Desorptionskolonne und kleiner als 6 bar arbeitet, durch indirekten Wärmeaustausch beheizbar ist und am Kopf einen Gasstrom abgibt, der im wesentlichen aus Wasserdampf besteht, welcher in die Ethylenoxid-Desorptionskolonne, aus der das glykolhaltige Wasser stammt, als Desorptionsstrom geleitet wird, wobei die bereitzustellende Wärmemenge nicht größer als die in dem Fall gelieferte ist, in dem das erfindungsgemäe Verfahren nicht angewendet und Ethylenglykol in bekannter Weise vom am unteren Ende der Kolonne erhaltenen Gemisch durch Entfernung des Wassers und Verdampfung gewonnen wird.

2. Verfahren nach Anspruch 1, dadurch gekennzeichnet, daß das glykolhaltige Wasser in die Ethylenglykol-Trennkolonne mit der Temperatur eingeleitet wird, mit der es die Ethylenoxid-Desorptionskolonne verläßt.

**Claims**

1. A process for separating ethylene glycol from glycolated water which issues from the bottom of an ethylene oxide desorption column in a process for manufacturing ethylene oxide by catalytic oxidation of ethylene in the vapour phase by oxygen, the glycolated water containing up to 10% ethylene glycol by weight and sodium or potassium salts derived from sodium or potassium hydroxide which has been added to the glycolated water to prevent corrosion by the carbonic acid or carboxylic acids which may be present, characterised in that the ethylene glycol is separated in the form of a mixture with water, containing 40% to 90% ethylene glycol by weight and the said salts, obtained at the bottom of an ethylene glycol separation column, the upper level of said column being fed directly with the glycolated water, the ethylene glycol separation column comprising below this upper level up to 12 theoretical plates and operating at a mean absolute pressure at least equal to that of the said ethylene oxide desorption column, the mean absolute pressure being less than 6 bar, the ethylene glycol separation column being heated by indirect heat exchange, and producing at the top of the column a gaseous stream consisting substantially of steam which is introduced into the ethylene oxide desorption column from which the glycolated water issues for use as the desorption stream, the quantity of thermal energy to be supplied being no greater than that supplied when the process according to the invention is not employed, and the ethylene glycol being isolated from the mixture collected at the bottom of the column in known manner by elimination of water and evaporation.

2. A process according to claim 1, characterised in that the glycolated water is introduced into the ethylene glycol separation column at the temperature at which it issues from the ethylene oxide desorption column.